(19) Europäisches Patentamt
European Patent Office
Office européen des brevets

(11) **EP 4 491 098 A1**

(12) **EUROPEAN PATENT APPLICATION**

(43) Date of publication:
**15.01.2025  Bulletin 2025/03**

(21) Application number: **23306172.0**

(22) Date of filing: **10.07.2023**

(51) International Patent Classification (IPC):
***A61B 3/032*** (2006.01)    ***A61B 3/028*** (2006.01)

(52) Cooperative Patent Classification (CPC):
**A61B 3/028; A61B 3/032**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Essilor International**
**94220 Charenton-Le-Pont (FR)**

(72) Inventors:
• **BAROUD, Mira**
**94000 CRETEIL (FR)**

• **JAN, Hadrien**
**77500 CHELLES (FR)**
• **TRANVOUEZ BERNARDIN, Delphine**
**94000 CRETEIL (FR)**
• **BOUTINON, Stéphane**
**75013 PARIS (FR)**
• **ZHOU, Xinpei**
**SHANGHAI, 200052 (CN)**
• **SWITAL, Marion**
**75013 PARIS (FR)**

(74) Representative: **Korakis-Ménager, Sophie**
**Essilor International**
**Propriété Intellectuelle**
**147, rue de Paris**
**94220 Charenton-le-Pont (FR)**

(54) **DEVICE FOR DETERMINING A VISUAL BEHAVIOUR OF A PERSON**

(57)     Device for determining values of parameters representing the visual behaviour of a person, handheld digital device and computer-implemented method. The device is configured for determining a first visual acuity of the person by displaying at least one first optotype, a size of the at least one first optotype varying during the display according to a distance between the device and the person so that an angular size of the at least one first optotype, from a point of view of the person, is fixed. The device is also configured for determining a first relative motion, the first relative motion being a relative motion of the person with respect to the device during the determination of the first visual acuity.

[Fig. 1]

EP 4 491 098 A1

## Description

## Technical field

This disclosure relates to devices and handled digital devices for determining a visual behaviour of a person and computer-implemented methods for determining the visual behaviour.

## Background information and prior art

The determination of the visual behaviour of the person may be realized by Eye Care Professionals (ECP). As such, it requires setting up an appointment with an ECP which makes it difficult to regularly and frequently check the visual behaviour of the person.

The visual behaviour and values of parameters representing the visual behaviour may be determined based on several visual acuity tests carried out in different situations. Using different situations allows a more accurate determination of the visual behavior of the person compared to conventional visual acuity tests such as Snellen Chart.

Some methods of the prior art allow the determination of the visual acuity without requiring the intervention of an eye-care professional. However, none of these methods of the prior art allows the determination of the visual behaviour of the person.

Therefore, there is a need for a device and a method for determining the visual behaviour of a person that does not have the drawbacks of the devices and methods of the state of the art.

## Summary

The following presents a simplified summary to provide a basic understanding of various aspects of this disclosure. This summary is not an extensive overview of all contemplated aspects and is intended to neither identify key or critical elements of all aspects nor delineate the scope of any or all aspects. The sole purpose is to present some concepts of one or more aspects in a simplified form as a prelude to the more detailed description that is presented later.

One aspect of this disclosure is a device for determining a visual behaviour of a person. The device is configured for determining a first visual acuity of the person by displaying at least one first optotype, a size of the at least one first optotype varying during the display according to a distance between the device and the person so that an angular size of the at least one first optotype, from a point of view of the person, is fixed, the first visual acuity depending on a correctness of an indication, by the person, of a value of a parameter of the at least one first optotype, The device is also configured for determining a first relative motion, the first relative motion being a relative motion of the person with respect to the device during the determination of the first visual acuity. The device is also configured for determining a second visual acuity of the person by displaying at least one second optotype, a size of the at least one second optotype being fixed during the display of the at least one second optotype, the second visual acuity depending on a correctness of an indication, by the person, of a value of a parameter of the at least one second optotype. The device is also configured for determining a second relative motion, the second relative motion being a relative motion of the person with respect to the device during the determination of the second visual acuity. The device is also configured for determining the visual behaviour of the person based on the first visual acuity, the second visual acuity, the first relative motion and the second relative motion.

Another aspect of this disclosure is a handled digital device. The handled digital device is configured for determining a visual behaviour of a person. The handled digital devices is also configured for determining a first visual acuity of the person by displaying at least one first optotype, a size of the at least one first optotype varying during the display according to a distance between the device and the person so that an angular size of the at least one first optotype, from a point of view of the person, is fixed, the first visual acuity depending on a correctness of an indication, by the person, of a value of a parameter of the at least one first optotype, The handled digital device is also configured for determining a first relative motion, the first relative motion being a relative motion of the person with respect to the device during the determination of the first visual acuity. The device is also configured for determining a second visual acuity of the person by displaying at least one second optotype, a size of the at least one second optotype being fixed during the display of the at least one second optotype, the second visual acuity depending on a correctness of an indication, by the person, of a value of a parameter of the at least one second optotype. The device is also configured for determining a second relative motion, the second relative motion being a relative motion of the person with respect to the handled digital device during the determination of the second visual acuity. The device is also configured for determining the visual behaviour of the person based on the first visual acuity, the second visual acuity, the first relative motion and the second relative motion.

Another aspect of this disclosure is computer-implemented method for determining a visual behaviour of a person. The computer-implemented method comprises determining a first visual acuity of the person by displaying at least one first optotype, a size of the at least one first optotype varying during the display according to a distance between the device and the person so that an angular size of the at least one first optotype, from a point of view of the person, is fixed, the first visual acuity depending on a correctness of an indication, by the person, of a value of a parameter of the at least one first optotype, The computer-implemented method also comprises determining a first relative motion, the first relative motion being a relative motion of the person with respect to the device during the determination of the first visual acuity. The device is also configured for determining a second visual acuity of the person by displaying at least one second optotype, a size of the at least one second optotype being fixed during the display of the at least one second optotype, the second visual acuity depending on a correctness of an indication, by the person, of a value of a parameter of the at least one second optotype. The computer-implemented method also comprises determining a second relative motion, the second relative motion being a relative motion of the person with respect to the device during the determination of the second visual acuity. The computer-implemented method also comprises determining the visual behaviour of the person based on the first visual acuity, the second visual acuity, the first relative motion and the second relative motion.

A computer may include a memory and a processor. Examples of processors include microprocessors, microcontrollers, graphics processing units (GPUs), central processing units (CPUs), application processors, digital signal processors (DSPs), reduced instruction set computing (RISC) processors, systems on a chip (SoC), field-programmable gate arrays (FPGAs), programmable logic devices (PLDs), state machines, gated logic, discrete hardware circuits, and other suitable hardware configured to perform the various functionality described throughout this disclosure. The memory may be a computer-readable media. By way of example, and not limitation, such computer-readable media may include a random-access memory (RAM), a read-only memory (ROM), an electrically erasable programmable ROM (EEPROM), optical disk storage, magnetic disk storage, other magnetic storage devices, combinations of the aforementioned types of computer-readable media, or any other medium that may be used to store computer executable code in the form of instructions or data structures that may be accessed by the processor of the computer.

Another aspect of this disclosure is a non-transitory program storage device, readable by a computer, tangibly embodying a program of instructions executable by the computer to perform a method for determining values of parameters representing the visual behaviour of a person.

All the embodiments described for the device or the handheld digital device may be applied alone or in combination to the non-transitory program storage device.

## Description of the drawings

For a more complete understanding of the description provided herein and the advantages thereof, reference is now made to the brief descriptions below, taken in connection with the accompanying drawings and detailed description, wherein like reference numerals represent like parts.

Figure 1 represents a device for determining values of parameters representing a visual behaviour of a person.
Figure 2 represents Landolt C optotypes in various sizes and orientations.
Figure 3 represents an angular size of an object.
Figure 4 represents the method, determining values of parameters representing a visual behaviour of a person.
Figure 5 represents an example of the device for determining values of parameters representing a visual behaviour of a person.

## Detailed description of embodiments

**The** detailed description set forth below in connection with the appended drawings is intended as a description of various possible embodiments and is not intended to represent the only embodiments in which the concepts described herein may be practiced. The detailed description includes specific details for the purpose of providing a thorough understanding of various concepts. However, it will be apparent to those skilled in the art that these concepts may be practiced without these specific details. In some instances, well-known structures and components are shown in block diagram form to avoid obscuring such concepts.

### *Description of the device for determining values of parameters representing a visual behaviour of a person*

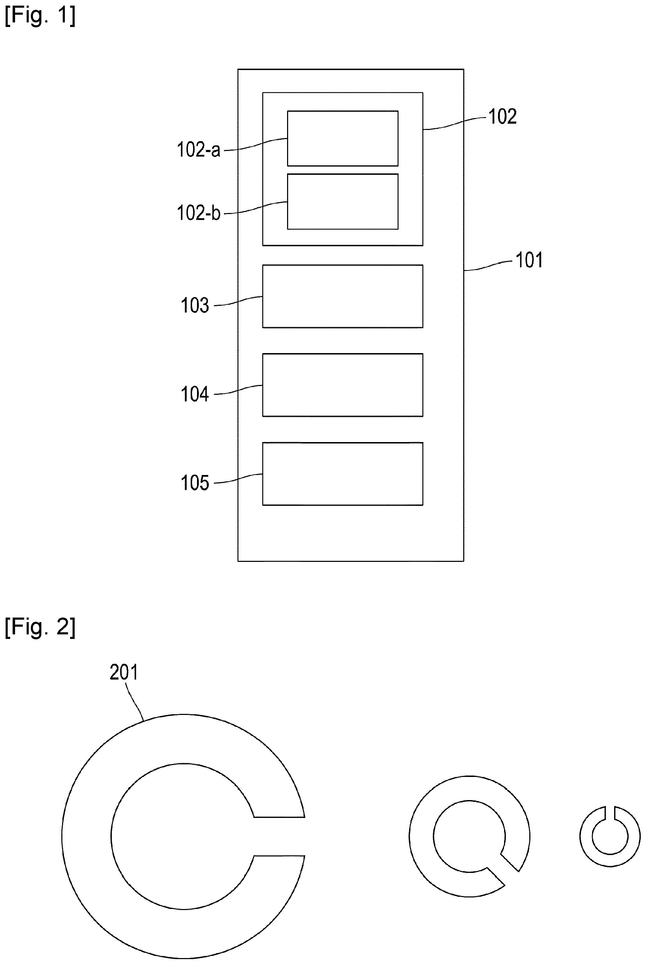

Figure 1 represents a device 101 for determining a visual behaviour of a person more precisely values of parameters representing the visual behaviour of the person. The device 101 comprises a calculation module 102. The calculation module 102 is configured to determine the values of the parameters representing the visual behaviour of the person.

The device 101 may be a handheld device for example a smartphone or a smart tablet.

The visual behaviour may be used to determine values of parameters representing values of parameters of optical lenses adapted to the person. These parameters may represent designs of the optical lenses, treatments of the optical lenses, a tint of the optical lenses and/or other parameters representing functionnalities of the optical lenses. The parameters may also comprise metrics on design and tint zones usage by the person. The parameters may also comprise metrics on the good or poor usage of design or tint. The usage may comprise the frequency of use of the different zones of the lenses, including the power use in zones. The parameters may also include facial landmarks to digitalize the visual behaviour in a digitwin of the person. The parameters may represents a state of the visual function including visual fatigue, poor visual correction, visual deficit evolution determined thanks to the visual behaviour.

The device 101 may also comprise a display unit 103. This display unit 103 may be connected to the calculation module 102 and may be use to give instructions to the person during the determination of the values of the parameters representing the visual behaviour of the person.

The display unit 103 may be a screen.

When the device 101 is a handled digital device the display unit 103 may be a screen of the handled digital device. The display unit 103 may have a resolution of 1440 pixels by 3040 pixels and a pixel density equal to 550 dpi. In this case a width of the display unit 103 is equal to 66.5mm and a height of the display unit 103 is equal to 140.4mm.

The display unit 103 may be configured to display optotypes.

The optotypes are standardised symbols, figures or letters of different sizes. The optotypes are used to test the visual acuity of the person.

The optotypes may be displayed one by one on the display unit 103. Alternatively, optotypes may be displayed line by line with multiple optotype on each line. The size and/or orientation and/or contrast of each optotype may vary during the displaying step. Alternatively, multiple lines of optotypes may be displayed at the same time, with optotypes of different size and/or orientation and/or contrast on each line. In this case the displayed optotypes may form a LogMAR chart (Logarithm of the Minimum Angle of Resolution). Examples of LogMAR charts are Bailey-Lovie chart and the ETDRS (Early Treatment Diabetic Retinopathy Study) charts.

The optotypes may be Landolt C or Snellen E. The Landolt C is also known as a Landolt ring, Landolt broken ring, or Japanese vision test. It was developed by the Swiss-born ophthalmologist Edmund Landolt. Figure 2 represents optotypes 201. The optotypes 201 of figure 2 are Landolt C in various sizes and orientations.

**The** device 101 may also comprise an input module 104 configured to obtain inputs from the person. The input module 104 may be for example a trackpad.

In embodiments the display unit 103 and the input module 104 may form a single module. For example when the device 101 is the handled digital device a touch screen of the handled digital device may form the display unit 103 and the input module 104.

**The** device 101 may comprise a distance module 105 configured to determine a distance between the person, especially the right eye, the left eye or the cyclopean eye and the display unit 103, especially an optotype when one optotype is displayed on the display unit 103. The distance may be determined between the pupil and the optotype.

The distance module 105 may advantageously be configured to determine the distance when the person is facing the display unit 103.

The distance module 105 may comprise at least one camera.

The distance module 105 may be configured to provide at anatomical landmarks of a face of the person. These anatomical landmarks for example anchor head points may be used to obtain the distance and a declination and a tilt of the face of the person with respect to the display unit 103.

The distance module 105 may be a TrueDepth sensor.

The device 101 may also comprise a parallelism determination module. The parallelism determination module may determine a parallelism between the display unit 103 and a plane sensibly parallel to a face of the person and possibly passing by the eyes of the person.

The device 101, especially the calculation module 102 and/or the display unit 103 may be configured to adapt a size of the displayed optotypes based on the measured distance and possibly the declination and the tilt of the face of the person and/or the declination and the tilt of the device 101.

In embodiments this adaptation may be realized so that an angular size of the displayed optotype, calculated from the face of the person for example the left eye, the right eye or the cyclopean eye, is constant whatever the measured distance.

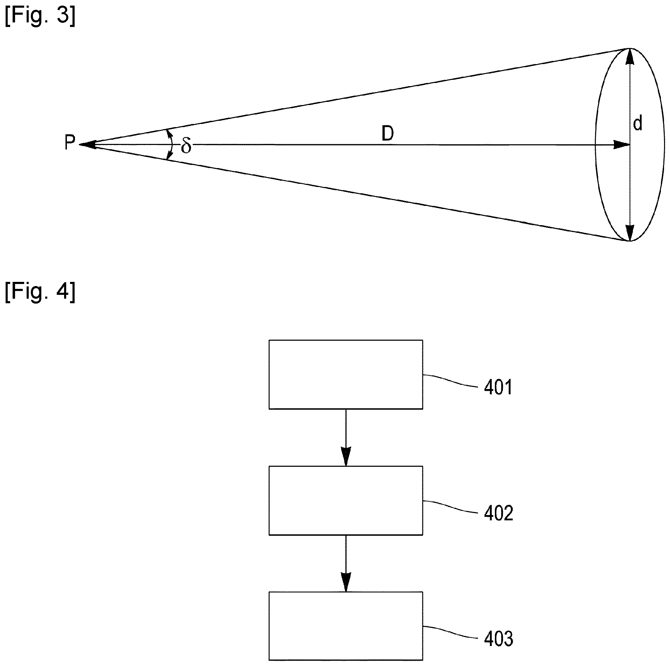

The angular size represents how large an object for example an optotype appears from a point P. In the example of figure 3, the distance D is the distance between the point P and the object, d is the size of the object and $\delta$ is the angular size of the object as seen from the point P. The angular size may be obtained using the

equation $\delta = 2\arctan\left(d/2D\right)$

In the following part of this description, the size of the optotype represents how large the optotype is as it is displayed on the screen. The size may be expressed in cm, mm or number of pixels of the optotype. The size of the optotype does not depend on the point of view.

In the following part of this description, the angular size represents how large the optotype is when this optotype is seen from a given point of view. The angular size is expressed in degree. The angular size of the optotype depend on the size of the optotype and the point of view of this optotype.

According to the previously presented equation, to have an optotype presenting a constant angular size, the size of the optotype, as displayed on the screen, must be reduced by a factor equal to a reducing factor of the measured distance when the distance is reduced.

The calculation module 102 comprises a memory 102-a and a processor 102-b.

The calculation module 102 may be a low resource calculation module.

Examples of processors 102-b include microprocessors, microcontrollers, graphics processing units (GPUs), central processing units (CPUs), application processors, digital signal processors (DSPs), reduced instruction set computing (RISC) processors, systems on a chip (SoC), baseband processors, field-programmable gate arrays (FPGAs), programmable logic devices (PLDs), state machines, gated logic, discrete hardware circuits, and other suitable hardware configured to perform the various functionality described throughout this disclosure.

The memory 102-a is computer-readable media. By way of example, and not limitation, such computer-readable media may include a random-access memory (RAM), a read-only memory (ROM), an electrically erasable programmable ROM (EEPROM), optical disk storage, magnetic disk storage, other magnetic storage devices, combinations of the aforementioned types of computer-readable media, or any other medium that may be used to store computer executable code in the form of instructions or data structures that may be accessed by the processor 102-a of the calculation module 102.

In embodiments, the calculation module 102 may be included in independent module, for example, a smartphone or a computer, a system on chip (SoC) or a graphics processing unit (GPU). The calculation module 102 may also be a virtual machine located on a cloud network or a server not co-located with the patient or the system 101.

The device 101 may further comprise physical plus and minus volume buttons. Preferably, the plus volume button and the minus volume button are disposed on the side of the device 101, for example on the left side of the device 101 when facing the display unit 103. The plus and minus volume buttons may be used by the person to provide indications during the determination of the values of the parameters representing the visual behaviour of the person.

The device 101 may comprise an inertial measurement unit (IMU) configured to obtain an indication of a motion or a declination of the device 101 while the determination of the values of the parameters representing the visual behaviour of the person. The inertial measurement unit may also enable to quantify values of parameters representing the motion of the person. For example, the inertial measurement unit may also determine values of parameters representing the walking of the person or gestures of the person. The parameters representing the walking may comprise a walking cadence.

The visual behaviour may be determined based on implicit measures and explicit measures.

The implicit measures may be:

- The distance between the right eye, the left eye, the cyclopean eye and the display unit 103 of the device 101.
- The device 101 closest distance rate. By the device closest distance rate, one means the number of times the person tried to bring the device closer to their face. For example, one may measure the number of times the distance between the face of the person and the device 101 reaches the TrueDepth sensor accuracy limit (about 22cm).

- Results on the visual acuity tests of the person in different daily situations, for example the best visual acuity of the person.
- The best dynamic visual acuity of the person in different dynamic situations : walking, cycling, standing, ...and in different ambient light environment.
- The blink rate and the eye lid aperture of the person.
- The gaze direction of the person related to the device 101, especially the display unit 103.
- The walking cadence of the person.
- The position and the motion of the device 101, especially the display unit 103, absolute and relative to the head of the person.
- The orientation and angular motion of the device 101, especially the display unit 103, absolute and relative to the head of the person and
- Values of parameters representing the ambient light.

The explicit measures may be:

- Rating of visual fatigue obtained from a previous examination of the person,
- Rating of blurred or double vision experienced by the person,
- Rating of lens discomfort experienced by the person,
- Free comments of the person regarding its visual state such as fatigue, blurred vision, etc..

### Description of the method for determining values of parameters representing a visual behaviour of a person

To realise this determination of the visual behaviour of the person and possibly the values of the parameters representing the visual behaviour of the person, the memory 102-a may store a computer program comprising instructions which, when the program is executed by the processor 102-b, cause the control module 102 to carry out a method, for example a computer-implemented method, for determining the visual behaviour of the person and possibly the values of the parameters representing the visual behaviour of the person. The method, as presented in figure 4, comprises:

- a step 401 of determining a first visual acuity of the person by displaying at least one first optotype, the size of the at least one first optotype varying during the display according to a distance between the device 101 and the person, so that an angular size of the at least one first optotype, from a point of view of the person, is fixed, the first visual acuity depending on a correctness of an indication, by the person, of a value of a parameter of the at least one first optotype,
- a step 402 of determining a second visual acuity of the person by displaying at least one second optotype, the size of the at least one second optotype being fixed during the display of the optotype, and the second visual acuity depending on a correctness of an indication, by the person, of a value of a parameter of the at least one second optotype,
- a step 403 of determining the visual behaviour of the person based on the first visual acuity and the second visual acuity.

The method of the figure 4 may also comprise a determination of a first relative motion of the face of the person, for example the left eye, the right eye or the cyclopean eye, with respect to the device 101 during the determination of the first visual acuity.

The method of the figure 4 may also comprise a determination of first distances between the face of the person, for example the left eye, the right eye or the cyclopean eye, and the device 101 during the determination of the first visual acuity.

**The** method of the figure 4 may also comprise a determination of a second relative motion of the face of the person, for example the left eye, the right eye or the cyclopean eye, with respect to the device 101 during the determination of the second visual acuity.

**The** method of the figure 4 may also comprise a determination of second distances between the face of the person, for example the left eye, the right eye or the cyclopean eye, and the device 101 during the determination of the second visual acuity.

By relative motion of a first object with respect to a second object, one means the motion of the first object in a referentiel in which the second object is fixed.

The visual behaviour of the person may also be determined based on the first relative motion, the first distances, the second relative motion, and/or the second distances.

In embodiments when during the step 401 the distance is out of a predetermined range, the method may comprise the display, using the display unit 103, of an indication toward the person to move the device 101 in the predetermined distances range. This avoid the person being too close or too far of the device 101 and therefore reaching the resolution limit of the display unit 103 of the device 101.

The visual acuity may refer to the clarity of vision, but technically rates an ability of the person to recognize details with small angular size with precision.

In this disclosure the visual acuity may also be determined when the face of the person has a motion relative to the device 101. The visual acuity may be evaluated for a person with or without his glasses.

To determine the visual acuity of a person a plurality of optotypes, described previously, may be presented to the person and the person must recognize a value of a parameter of the optotypes. The optotypes may be presented with a different size and the visual acuity is determined based on the correctness of the recognition of the value by the person.

During the step 401 or the step 402 a series of various randomized optotypes may be presented to the person. The indications received from the person are evaluated and a first visual acuity and a second visual acuity, corresponding to the correctness of the recognition, by the person, of the value of the parameters of the presented optotypes, are determined. In this case the visual acuity may be determined based on the correctness of the recognition of the last displayed optotype or based on the optotype allowing a correctness of the recognition of about 60%.

More precisely during the step 401 of determining the first visual acuity, a plurality of first optotypes may be displayed to the person. The first optotypes may be displayed one after the other. The person may reply to a query regarding a value of a parameter of the display optotype. When the displayed optotypes are Landolt C with an aperture having different directions, the person may indicate the direction of the aperture he perceived. The direction of the aperture of the Landolt C may be the parameter of this optotype. After the indication of the direction by the person a subsequent optotype is displayed. The subsequent optotype may have the same type as the previously displayed optotype. If the plurality of the first optotypes comprise Landolt C the subsequent optotype displayed is a Landolt C. The angular size of the subsequent optotype may depend on a correctness of the recognition of the value of the parameter of at least the previous optotype and possibly of a probability of this correctness of the recognition.

More precisely during the step 402 of determining the first visual acuity, a plurality of second optotypes may be displayed to the person. The second optotypes may be displayed one after the other. The person may reply to a query regarding a value of a parameter of the display optotype. When the displayed optotypes are Landolt C with an aperture having different directions, the person may indicate the direction of the aperture he perceived. The direction of the aperture of the Landolt C is the parameter of this optotype. After the indication of the direction by the person a subsequent optotype is displayed. The subsequent optotype may have the same type as the previously displayed optotype. If the plurality of the first optotypes comprise Landolt C the subsequent optotype displayed is a Landolt C. The anglar size of the subsequent optotype may depend on a correctness of the indication regarding the value of the parameter of the previous optotype.

The difference between the step 401 of determining the first visual acuity and the step 402 of determining the second visual acuity is the way the size of the displayed first or second optotype is adapted during this display.

During the step 401 the size of the first optotype is adapted dynamically so that the angular size of the first optotype, from the point of view of the person, is constant. i.e., when the distance between the person and the displayed first optotype is reduced the size of the optotype is reduced by a factor equal to the factor of reduction of the distance. The person may realise the first acuity test by placing the device 101 at a distance he is the most comfortable with.

On other words, the step 401 of determining the first visual acuity of the person is realized by displaying, on the display unit 103 of the device 101, successively a plurality of optotypes, at least one optotype of the plurality having a size depending on a size of a previous optotype and a correctness of a reply of the person regarding a parameter of the previous optotype, the size of the optotype varying according to a distance between the device and the person.

In other words, the step 402 of determining a second visual acuity of the person is realized by displaying, on the display unit 103 of the device 101, successively a plurality of optotypes, at least one optotype of the plurality having a size depending on a size of a previous optotype and a correctness of a reply of the person regarding a parameter of the previous optotype, the size of the optotype being fixed during the display of the optotype.

The first visual acuity may also be defined as the active visual acuity. In other words, during the determination of the active visual acuity one modulate the size of the optotype in real-time according to the distance between the device 101 and the person (for example the head or the left or right pupil of the person) to maintain the angular size, minimum angle resolution of the optotype and so improve the accuracy of the tested visual acuity tested. Using this visual acuity test one can determine also the minimum angle resolution at the most confortable holding distance of the handle device. This holding distance represents the minimum effort of accommodation and convergence and avoids the appearance of any visual compensatory behaviour when the optotype angular size decreases. It enables the determinations of the best dynamics visual acuity. During this test the person may be required to realize other activities like walking.

The second visual acuity is also known as the behavioural visual acuity. In this test the size of the displayed optotype is not adapted to the distance between the face of the person and the device 101. However, one may determine and record the evolution of this distance, or other parameters as the relative motion between the person and the device 101. During this test the person may be required to realize other activities like walking.

For both the step 401 and the step 402, the size of the successively displayed optotype may be selected using the staircase method developed by Standford University and described in the article "The Stanford Acuity Test: A Precise Vision Test Using Bayesian Techniques and a Discovery in Human Visual Response" Chris Piech et. al..

In embodiments the size of a given optotype may be smaller than the size of the previous optotype when the person correctly replies to the value of the parameter of the previous optotype.

In embodiments the size of a given optotype may be bigger than the size of the previous optotype when the person incorrectly replies to the value of the parameter of the previous optotype.

In embodiments the method may also comprise a step of determining a third visual acuity of the person. This third visual acuity is determined by displaying, on the display unit 103 of the device 101, successively a plurality of optotypes, at least one optotype having a size depending on a size of a previous optotype and a correctness of a reply of the person regarding a value of a parameter of the previous optotype, the size of the optotype being fixed during the display of the optotype.

This third visual acuity is also known as the static visual acuity. In this test the person may be required to stay immobile.

In embodiments the visual behaviour of the person may also be determined using the third visual acuity of the person.

When the person uses the device 101 implementing the method of the figure 4 the person may determine his visual behaviour without the need of an Eye Care Professional (ECP) or with a online monitoring of an Eye Care Professional (ECP).

The visual behaviour may be used to determine values of parameters representing an adaptation of the lenses to the person or values of parameters of lenses adapted to the person.

During the step 401 and the step 402 the person has to recognize the displayed optotype and to indicate to the device 101 which optotype is displayed. The indication of the person may be received using a keyboard, for example a virtual keyboard displayed on the display unit 103 and/or buttons, for example virtual buttons displayed on the display unit 103, and/or speech recognition and/or gesture recognition and/or the movement of the device 101.

When the optotypes displayed on the display unit 103 are words or short sentences, the person may identify the letter or read the sentences. These indications received from the person may be obtained using speech recognition, for example through a microphone of the device 101.

When the optotypes displayed on the display unit 103 are Landolt C with an aperture having different directions, the person may indicate the direction of the aperture he perceived. The indication relating to the direction of the aperture perceived by the person may be obtained by speech recognition using a microphone of the device 101, device 101 movement using an IMU of the device 101 or the front camera of the device 101, and head movement and/or arm movement using a front camera of the device 101. The person may also indicate the determined direction, by using the physical plus and minus buttons to indicate this determined direction.

In other words, in embodiments, the device 101 may be configured for receiving from a user interface, for example a touch pad, the reply of the person regarding the value of the parameter of the displayed optotype.

In embodiments, the optotypes may be displayed during a predetermined duration, when the person does not reply during the predetermined duration one may consider that the person incorrectly replies to the value of the parameter of the optotype.

The predetermined duration depending on at least one among:

- a duration of reply to the previously displayed optotype and
- a blink duration or blink frequency of the eyes of the person while displaying the previously displayed optotype.

The visual behaviour may comprise at least one among:

- A difference between, the first visual acuity and the the second visual acuity.
- A difference between a value of a parameter of the first relative motion and a value of a parameter of the second relative motion.
- An effort of accommodation-convergence during the first visual acuity test.
- An effort of accommodation-convergence during the second visual acuity test.
- A comparison between these two efforts.
- A functional visual acuity of the person. The functional visual acuity of the person is a representation of the dynamic visual acuity of the person. This dynamic visual acuity is based on the relative movement of the device 101 in relation to the head in terms of residual angular movement quantity (the movement of the device 101 not compensated by the eyes) and distance movement quantity.
- A visual fatigue of the person. Metrics related to visual fatigue of the person or/and the real time distance, motion, declination of the device 101 used by the person. Closest, upper, and more stable device 101 on the head/eye reference frame as well as the number of blinks of the person.
- A comfort holding distance of the person.
- An adaptation of eyeglasses worn by the person. To realize the determination of this adaptation, the first visual acuity and the second visual acuity of the person are determined when the person is wearing his eyeglasses and the person is not wearing his eyeglasses. By comparing the visual acuity obtained with the eyeglasses and without the eyeglasses one may determine if the eyeglasses, especially the design of the lenses is adapted to the person.
- A visual behaviour of the person may be based on a motion or a declination of the device 101 during the visual acuity tests. Closest, upper, and more stable device 101 on the head/eye reference frame as well as the amount of blink.

In embodiments the device 101 may be configured to obtain:

- values of parameters of an eye of the person,
- values of parameters representing a position or a behavior of the person,
- values of parameters of eyewear worn by the person and
- values of parameters representing an environment surrounding the person.

The visual behaviour may be also determined based on the previously described parameters.

The device 101 may be configured to display information representing the visual behaviour of the person. The device 101 may also be configured to display a reliability of the information representing the visual behaviour. The display of these elements may be realized on the display unit 103 of the device 101.

In embodiments the device 101 may be configured to determine visibility needs and contrasts sensitivity needs to have the best active visual acuity.

*Description of use-cases*

In a first use-case, the person may use a device 101, for example a smartphone. The device 101 comprises a distance module 105 for determining the distance between an optotype displayed by the device 101 and the person, more precisely the eye of the person.

The device 101 is configured to conduct multiple acuity tests. During one of the tests the third visual acuity, as described previously, is determined. To determine this third visual acuity several appearance of a single optotypes is displayed successively with the size varying according to an acuity staircase's algorithm. In this first test, the size of each optotype is fixed while the optotype is displayed on the screen of the device 101.

During a second test the first visual acuity, as described previously, is determined. As explained previously to determine the first visual acuity, the size of each optotype varies while the optotype is displayed on the screen to have a constant angular size of the optotype with respect to the point of view of the person. The angular size may be calculated with respect to the eye of the person.

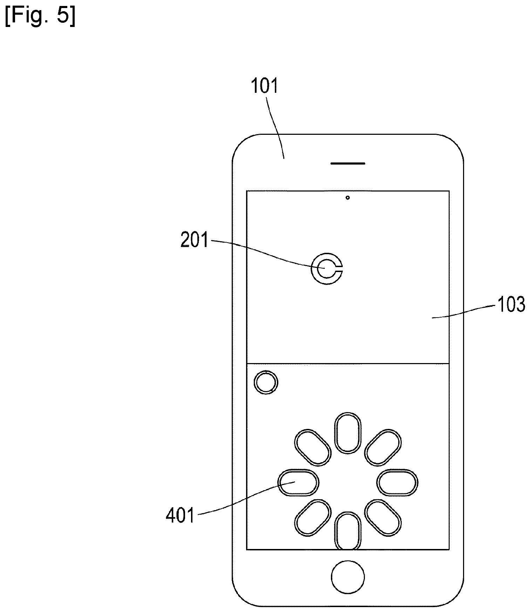

During the visual acuity tests, the person may use a touchpad integrated to the device 101 to answer a question regarding a value of a parameter of the optotype. This parameter may be the orientation of the optotype. The figure 5 represents the device 101 (in the case of the figure 5 a smartphone) on which an optotype 201 is displayed. On the bottom part of the display unit 103, a plurality of buttons 501 forming a touchpad is displayed. Using the buttons 501, the person may indicate to the device 101 what he recoginzes is the correct orientation of the optotype 201.

**The** device 101 may comprise sensors to measure the behaviour of the person and values of parameters of the environment surrounding the person during the visual acuity tests.

Once the visual behaviour of the person is determined the device 101 is configured to inform the person regarding his visual behaviour.

**The** device 101 displays successively different optotypes. The duration of display of each of the optotypes may depend on the time that the person needed to response to the previous optotype or parameters of the blinking of the person while the previous optotype was displayed.

The device 101 may also obtain (prior to the tests) data on the refraction and binocular profile of the person (accommodation max, lag of accommodation, proximal point of convergence ...), symptoms of visual fatigue, design of the lens used by the person, tint of the lens used by the person.

The device 101 may use the result of the visual acuity test and the previously described parameters to determine:

- An effort of accommodation-convergence during the second visual acuity test.
- An effort of accommodation-convergence during the first visual acuity test.
- A comparison between these two efforts.
- The active visual acuity results related to the static visual acuity as a function of the number of active situations (motion of the device 101, a motion of the device 101 relative to a motion of the head, retinal image stabilization behaviour). This allows the assessment of the dynamic visual acuity. This dynamic visual acuity is based on the relative movement of the device 101 in relation to the head in terms of residual angular movement quantity (the movement of the device 101 not compensated by the eyes) and distance movement quantity.
- Metrics related to visual fatigue of the person or/and the real time distance, motion, declination of the device 101 used by the person. Closest, upper, and more stable device 101 on the head/eye reference frame as well as the number of blinks of the person.
- The comfort holding distance of the person.

In addition to these different parameters related to the visual behaviour of the person, the device 101 may also provide information to an adaptation of the lenses used by the person. For example, the device 101 may provide design/tint ergonomics behaviour at least metrics related to the design or filter, at least metrics on design and tint zone usage, frequency, at least anatomical landmarks reference frame to digitalize the recorded behavior and compute new design based on distance, declination and tilt.

For the different information provided by the device 101, an indication of the reliability of this information, more precisely of the reliability of the visual acuity and the comfort holding distance of the person, may also be provided.

In a second use-case, one may compare the first visual acuity and the second visual acuity. During the second visual acuity test the person may not place the device 101 as he wishes, indeed when the device 101 is not a distance with a specified range the optotype is not displayed. Therefore, when the second visual acuity is lower than the first visual acuity it probably indicates that the person is developing visual fatigue. People developing visual fatigue will hold closer the device 101, decrease visual acuity, hold higher the device 101 in the visual field, and have shorter time responses at the staircase. For example, an decrease of visual acuity of 0.05 LogMAR between the first visual acuity and the second visual acuity may indicate that the person is developing visual fatigue.

In a third use-case, the device 101 may also be configured to determine the visual acuity target depending upon the motor activity of the person and the ability to stabilize the image displayed by the device 101 on the retina of the person.

In a fourth use-case, the device 101 may also be configured to measure zones of the lens that are used during near vision zone and frequency of this usage. This information may be used to determine visual fatigue especially when the person is a young non presbyopic, a presbyopic or a ametropic.

**Claims**

1. Device (101) for determining a visual behaviour of a person,
   the device being configured for:

   - determining a first visual acuity of the person by displaying at least one first optotype (201), a size of the at least one first optotype (201) varying during the display according to a distance between the device and the person, so that an angular size of the at least one first optotype (201), from a point of view of the person, is fixed,
   - determining a first relative motion, the first relative motion being a relative motion of the person with respect to the device (101) during the determination of the first visual acuity,
   - determining a second visual acuity of the person by displaying at least one second optotype, a size of the at least one second optotype (201) being fixed during the display of the at least one second optotype (201),
   - determining a second relative motion, the second relative motion being a relative motion of the person with respect to the device (101) during the determination of the second visual acuity and
   - determining the visual behaviour of the person based on the first visual acuity, the second visual acuity, the first relative motion and the second relative motion.

2. The device (101) according to the claim 1, the visual behaviour comprising at least one among:

- a difference between the first visual acuity and the second visual acuity,
- a difference between a value of a parameter of the first relative motion and a value of a parameter of the second relative motion and
- a visual fatigue of the person.

3. The device (101) according to the claim 1 or 2, the distance being a distance between the right eye or the left eye of the person and the at least one first optotype (201).

4. The device (101) according to any one of the claims 1 to 3, when determining the first visual acuity:

- the angular size of the at least one first optotype being smaller than an angular size of a previously displayed optotype when the person correctly replies to a value of a parameter of the previously displayed optotype and
- the angular size of the at least one first optotype being bigger than the angular size of the previously displayed optotype when the person incorrectly replies to the value of the parameter of the previously displayed optotype,

and/or when determining the second visual acuity:

- the size of the at least one second optotype being smaller than a size of a previously displayed optotype when the person correctly replies to a value of a parameter of the previously displayed optotype and/or
- the size of the at least one second optotype being bigger than the size of the previously displayed optotype when the person incorrectly replies to the value of the parameter of the previously displayed optotype.

5. The device (101) according to any one of the claims 1 to 4,

the at least one first optotype being a Landolt C and the parameter of the at least one first optotype being an orientation of the at least one first optotype and/or the at least one second optotype being a Landolt C and the parameter of the at least one second optotype being an orientation of the at least one second optotype.

6. The device (101) according to any one of the claims 1 to 5, the visual behaviour being also determining based on at least one of:

- values of parameters of an eye of the person,
- values of parameters representing a position
- values of parameters representing a behavior of the person,
- values of parameters of eyewear worn by the person and
- values of parameters representing an environment surrounding the person.

7. The device (101) according to any one of the claims 1 to 6,

the at least one first optotype being displayed during a first predetermined duration, when the person does not reply during the first predetermined duration one considers that the person incorrectly replies to the value of the parameter of the at least one first optotype and/or the at least one second optotype being displayed during a second predetermined duration, when the person does not reply during the second predetermined duration one considers that the person incorrectly replies to the value of the parameter of the at least one second optotype.

8. The device (101) according to the claim 6, the first predetermined duration and/or the second predetermined duration depending on at least one among:

- a duration of reply to a previously displayed optotype and
- a blink duration or blink frequency of the person while displaying the previously displayed optotype.

9. The device (101) according to any one of the claims 1 to 8, the device (101) being configured for receiving from a distance module (105) the distance.

10. The device (101) according to any one of the claims 1 to 9, the device (101) being configured for receiving from a user interface, for example a touch pad, the reply of the person.

11. The device (101) according to any one of the claims 1 to 10, the device (101) being configured for displaying the visual behaviour.

12. The device (101) according to any one of the claims 1 to 11,
the device (101) being configured for determining and possibly displaying a reliability of the visual behaviour.

13. The device (101) according to any one of the claims 1 to 12,
the device (101) being configured for determining visual needs and contrast sensitivity needs for the person to have a best active visual acuity.

14. Handled digital device, the handled digital device being configured for determining a visual behaviour of a person,
the handled digital device being also configured for:

- determining a first visual acuity of the person by displaying at least one first optotype (201), the size of the at least one first optotype (201) varying during the display according to a distance between the device and the person, so that an angular size of the at least one first optotype (201), from a point of view of the person, is fixed,
- determining a first relative motion, the first relative motion being a relative motion of the person with respect to the handled digital device during the determination of the first visual acuity,
- determining a second visual acuity of the person by displaying at least one second optotype, the size of the at least one second optotype (201) being fixed during the display of the at least one second optotype (201),
- determining a second relative motion, the second relative motion being a relative motion of the person with respect to the handled digital device during the determination of the second visual acuity and
- determining the visual behaviour of the person based on the first visual acuity, the second visual acuity, the first relative motion and the second relative motion.

15. Computer-implemented method for determining a visual behaviour of a person,
the computer-implemented method comprising:

- determining (401) a first visual acuity of the person by displaying at least one first optotype (201), the size of the at least one first optotype (201) varying during the display according to a distance between the device and the person, so that an angular size of the at least one first optotype (201), from a point of view of the person, is fixed,
- determining a first relative motion, the first relative motion being a relative motion of the person with respect to the device (101) during the determination of the first visual acuity,

- determining (402) a second visual acuity of the person by displaying at least one second optotype, the size of the at least one second optotype (201) being fixed during the display of the at least one second optotype (201),
- determining a second relative motion, the second relative motion being a relative motion of the person with respect to the device (101) during the determination of the second visual acuity and
- determining (403) the visual behaviour of the person based on the first visual acuity, the second visual acuity, the first relative motion and the second relative motion.

[Fig. 1]

102-a

102

102-b

101

103

104

105

[Fig. 2]

201

[Fig. 3]

[Fig. 4]

[Fig. 5]

Europäisches
Patentamt

European
Patent Office

Office européen
des brevets

# EUROPEAN SEARCH REPORT

Application Number

EP 23 30 6172

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/195951 A1 (LIMON OFER [IL]; ANCRI OFER KORN [IL]; ZLOTNIK ALEXANDER [IL]) 11 December 2014 (2014-12-11) | 1-6, 9-11, 13-15 | INV. A61B3/032 A61B3/028 |
| Y | * paragraph [0009] - paragraph [0283]; figures 1-27B * | 7,8,12 | |
| Y | IT VR20 120 111 A1 (DUEFFE TECNOVISION S A S DI FANTON FRANCO & C) 1 December 2013 (2013-12-01) * page 3, lines 2-8 * | 7 | |
| Y | VERSTEEG A BEGEN CISCO SYSTEMS T VANCAENEGEM ALCATEL-LUCENT Z VAX MICROSOFT CORPORATION B: "Unicast-Based Rapid Synchronization with RTP Multicast Sessions; draft-versteeg-avt-rapid-synchronization-for-rtp-02.txt", UNICAST-BASED RAPID SYNCHRONIZATION WITH RTP MULTICAST SESSIONS; DRAFT-VERSTEEG-AVT-RAPID-SYNCHRONIZATION-FOR-RTP-02.TXT, INTERNET ENGINEERING TASK FORCE, IETF; STANDARDWORKINGDRAFT, INTERNET SOCIETY (ISOC) 4, RUE DES FALAISES CH- 1205 GENEVA, SWITZE, no. 2, 9 March 2009 (2009-03-09), XP015061956, [retrieved on 2009-03-09] * page 181, paragraph 6.4 * | 8 | |
| Y | KR 2010 0104330 A (UNIV KWANGWOON IND ACAD COLLAB [KR]) 29 September 2010 (2010-09-29) * claim 1 * | 12 | |

TECHNICAL FIELDS SEARCHED (IPC)

A61B

The present search report has been drawn up for all claims

| Place of search | Date of completion of the search | Examiner |
|---|---|---|
| Munich | 14 December 2023 | Mäki-Mantila, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 30 6172

This annex lists the patent family members relating to the patent documents cited in the above-mentioned European search report.
The members are as contained in the European Patent Office EDP file on
The European Patent Office is in no way liable for these particulars which are merely given for the purpose of information.

14-12-2023

| Patent document cited in search report | Publication date | Patent family member(s) | Publication date |
|---|---|---|---|
| WO 2014195951 A1 | 11-12-2014 | AU 2014276340 A1 | 24-12-2015 |
| | | BR 112015030406 A2 | 25-07-2017 |
| | | CA 2914456 A1 | 11-12-2014 |
| | | CN 105764405 A | 13-07-2016 |
| | | CN 110251066 A | 20-09-2019 |
| | | DK 3003121 T3 | 16-08-2021 |
| | | EP 3003121 A1 | 13-04-2016 |
| | | ES 2886136 T3 | 16-12-2021 |
| | | JP 6453859 B2 | 16-01-2019 |
| | | JP 6612959 B2 | 27-11-2019 |
| | | JP 2016523132 A | 08-08-2016 |
| | | JP 2019069180 A | 09-05-2019 |
| | | KR 20160017079 A | 15-02-2016 |
| | | PT 3003121 T | 06-09-2021 |
| | | RU 2706372 C1 | 18-11-2019 |
| | | RU 2015152290 A | 14-07-2017 |
| | | US 2016120402 A1 | 05-05-2016 |
| | | US 2017079523 A1 | 23-03-2017 |
| | | US 2019307324 A1 | 10-10-2019 |
| | | WO 2014195951 A1 | 11-12-2014 |
| IT VR20120111 A1 | 01-12-2013 | | |
| KR 20100104330 A | 29-09-2010 | NONE | |

EPO FORM P0459

For more details about this annex : see Official Journal of the European Patent Office, No. 12/82

**REFERENCES CITED IN THE DESCRIPTION**

**Non-patent literature cited in the description**

- **CHRIS PIECH**. *The Stanford Acuity Test: A Precise Vision Test Using Bayesian Techniques and a Discovery in Human Visual Response*